# EUROPEAN PATENT APPLICATION

(11) **EP 2 998 392 A1**
(43) Date of publication of application: **23.03.2016**
(21) Application number: 14184998.4
(22) Date of filing: 16.09.2014
(51) Int. Cl.: C12N 5/077, C12N 5/0775

(54) **A population of cells for use in single-stage cell-based cartilage regeneration**

(71) Applicant: The Provost, Fellows, Foundation Scholars, & the other members of Board, of the College of the Holy & Undiv. Trinity of Queen Elizabeth near Dublin, Dublin 2 (IE)
(72) Inventor: Kelly, Daniel, Dublin, D2 (IE); Buckley, Conor, Dublin, D2 (IE); Almeida, Henrique, Dublin, D2 (IE); Eswaramoorthy, Rajalakshmanan, Dublin, D2 (IE)
(74) Representative: Purdy, Hugh Barry

(57) **Abstract**

An isolated population of non-expanded cells derived from adipose tissue of a mammal and enriched in non-expanded chondro-progenitor cells is described.

## Description

### Background to the Invention

In humans, 95% of defects to the articular surface of synovial joints involve cartilage without affecting the subchondral bone (Hjelle et al., 2002). Such defects fail to heal spontaneously. An estimated 5.4 million patients in the US alone will require joint and cartilage procedures to treat such defects and other degenerative changes by 2019. Bone marrow stimulation techniques such as microfracture are the most readily available clinical repair strategies for articular cartilage (Getgood et al., 2009). By surgically penetrating the subchondral bone, progenitor cells from the bone marrow can migrate into the defect and form a repair tissue. In general, a mechanically inferior fibro-cartilaginous tissue is produced which provides only temporary symptomatic relief. Alternative cell based therapies such as autologous chondrocytes implantation (ACI) are available (described in detail below), however these approaches require two hospital stays and are very expensive (∼€35,000), which may explain their relatively limited clinical uptake compared to marrow stimulation techniques. The surgical choice facing clinicians is further complicated by the fact that ACI performed after failed bone marrow stimulation techniques have a significantly higher failure rate and inferior clinical outcomes when compared with ACI as a first-line of defence treatment (Pestka et al., 2012; Minas et al, 2009). Therefore, there is a significant commercial opportunity for a cost effective 'single-stage' or 'in-theatre' therapy for regenerating damaged articular cartilage that does not necessitate damaging the subchondral bone. Such a therapy would represent a first line treatment option for damaged articular cartilage.

Delivery of isolated chondrocytes into cartilage defects, a technique known as autologous chondrocyte implantation (ACI), is a promising strategy for filling defects with in situ forming cartilaginous tissue, and has been in clinical use for a number of years. The technique is implemented in two stages. During the first intervention, chondrocytes are obtained from cartilage biopsies taken from low-load bearing regions of the joint and are culture expanded in vitro. During the second intervention, the expanded chondrocytes are injected into the defect, which is sealed with a sutured periosteal flap or collagen membrane. The results after 9 years follow-up indicate a durable symptomatic relief and partial structural repair (Peterson et al., 2000). Variations on ACI, such as matrix assisted chondrocyte implantation (MACI), whereby cells are seeded on a scaffold prior to implantation, have also been in clinical trials/use for a number of years. MRI assessment of cartilage repair has revealed a more hyaline-like zonal architecture following MACI compared to microfracture (Welsch et al., 2008) - the standard clinical procedure performed to treat cartilage defects. While MACI is technically attractive, and may allow larger defects to be treated, it has yet to be established if clinical outcomes following MACI are superior to ACI (Bartlett, 2005). Furthermore, both techniques require two operations and a prolonged culture period before implantation making them prohibitively expensive. The repair tissue is still at best hyaline-like.

It is an object of the invention to overcome at least one of the above-referenced problems.

### Statements of Invention

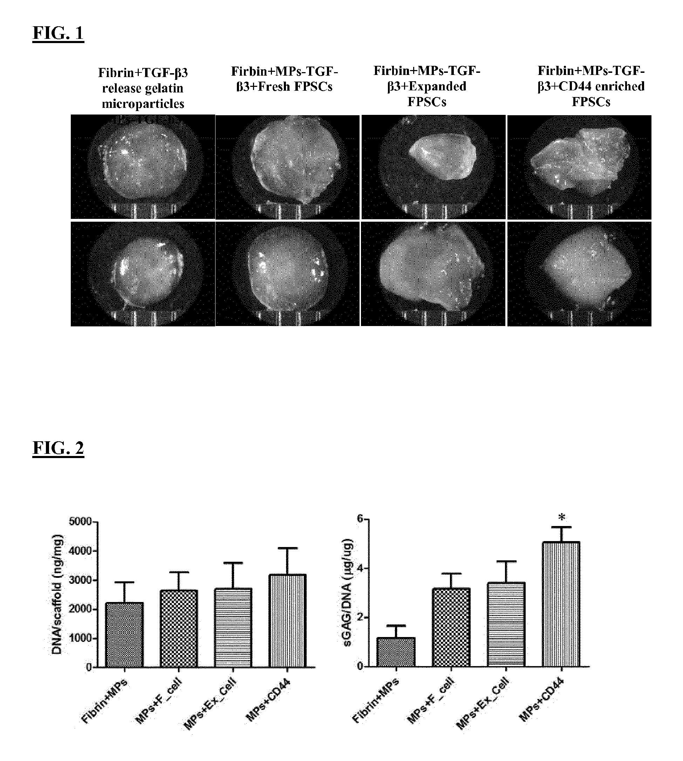

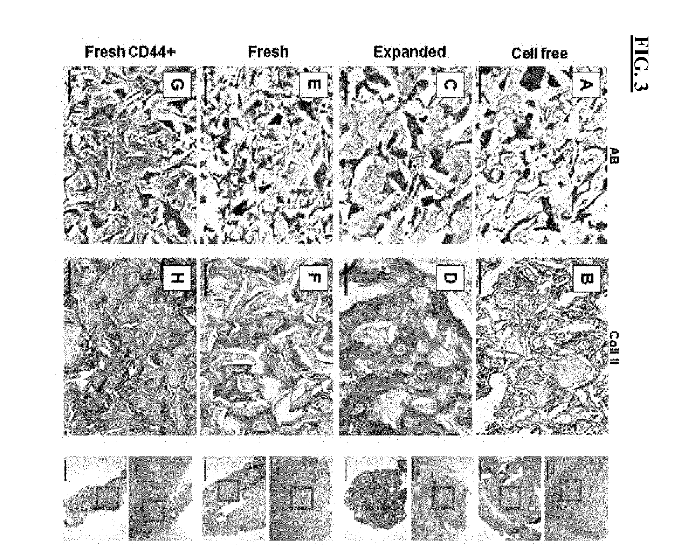

The invention provides a non-expanded chondro-progenitor cell or cell population derived from adipose tissue, particularly joint derived adipose tissue or synovial tissue, and ideally infrapatellar fat pad tissue (also know as the Hoffas fat pad), of a mammal, and the use of the cells for *in-vivo* articular cartilage regeneration. The cell population of the invention has been found to have significant chondrogenic potential and sufficient cellularity to enable cartilage repair within the context of a one-step surgical procedure. Specifically, the *in-vivo* data presented below shows that the cells of the invention, when seeded into two different scaffolding systems (fibrin hydrogel and cartilage ECM-derived scaffold), resulted in at least comparable cartilage formation compared with culture expanded stromal cell populations (Figs. 1 and 3), and significantly higher sGAG synthesis compared with either fresh (unsorted) or expanded stromal cell populations (Fig. 2, 4 and 5).

In the first instance, the invention provides a non-expanded chondro-progenitor cell derived from adipose tissue.

Typically, the non-expanded chondro-progenitor cell is CD44 or CD271 positive.

Preferably, the non-expanded chondro-progenitor cell is derived from joint adipose tissue or synovial tissue, ideally from the infrapatellar fat pad tissue.

In another aspect, the invention relates to an isolated population of non-expanded cells derived from adipose tissue of a mammal and enriched in non-expanded chondro-progenitor cells.

Typically, the non-expanded chondro-progenitor cells are CD44 or CD271 positive.

Preferably, the non-expanded chondro-progenitor cells are derived from joint adipose tissue, ideally infrapatellar fat pad tissue.

In another aspect, the invention relates to an isolated population of chondro-progenitor cells obtained by the steps of isolating stromal cells from adipose tissue without expansion of the cells, enriching the non-expanded stromal cells for cells that exhibit a cell surface marker associated with stem cells, specifically CD44 or CD271, to provide a population of non-expanded chondro-progenitor cells.

The invention also relates to a composition comprising cells of the invention (or an isolated population of cells of the invention) in combination with a second different cell type, for example freshly isolated chondrocytes or chondrons, optionally including at least one further component selected from a biological growth factor and extracellular matrix material.

The invention also relates to a composition comprising cells of the invention (or an isolated population of cells of the invention) in combination with a growth factor, for example TGF-β3, and optionally including at least one further component selected from with a second different cell type (for example freshly isolated chondrocytes or chondrons) and extracellular matrix material.

The invention also relates to a composition comprising cells of the invention (or an isolated population of cells of the invention) in combination with extracellular matrix material, typically micronized extracellular matrix, and optionally including at least one further component selected from a biological growth factor and freshly isolated chondrocytes or chondrons. The invention also relates to a bioactive tissue repair construct comprising a tissue repair construct seeded with cells of the invention, or an isolated population of cells of the invention, or a composition of the invention.

Typically, the tissue repair construct is derived from a hydrogel or a solid porous scaffold. Hydrogels and porous scaffolds suitable for use with the present invention are described below.

Suitably, the porous scaffold is configured for use in articular defect repair.

The invention also relates to a method for *in-vivo* repair of a defect site in cartilage in a patient comprising the steps of implanting a bioactive tissue repair construct of the invention at the defect site in the patient.

The invention also relates to a method for *in-vivo* repair of a defect site in cartilage in a patient comprising the steps of:
- isolating stromal cells from adipose tissue obtained from the patient without expansion of the cells;
- enriching the non-expanded stromal cells for cells that exhibit a cell surface marker associated with stem cells, to provide a population of non-expanded chondro-progenitor cells;
- seeding or combining a tissue repair construct matrix with the population of non-expanded chondro-progenitor cells to provide a bioactive tissue repair construct; and
- implanting the bioactive tissue repair construct in the defect site.

Typically, the cartilage is articular cartilage.

Suitably, the adipose tissue is joint adipose tissue, preferably joint fat pad adipose tissue, and ideally infrapatellar fat pad tissue.

Typically, the cell surface marker associated with stem cells is CD44 or CD271.

Preferably, the 3-D matrix is selected from a porous scaffold or a hydrogel.

In a preferred embodiment, the invention relates to a method for *in-vivo* repair of a defect site in articular cartilage in a patient comprising the steps of:
- isolating stromal cells from adipose tissue, preferably infrapatellar fat pad tissue, obtained from the patient without the traditional expansion of cells;
- enriching the non-expanded stromal cells for CD44 or CD271 positive cells to provide a population of non-expanded chondro-progenitor cells;
- seeding a tissue repair construct with the population of non-expanded chondro-progenitor cells to provide a bioactive tissue repair construct; and
- implanting the bioactive tissue repair construct in the defect site.

In a further embodiment, the invention relates to a method for *in-vivo* repair of a defect site in articular cartilage in a patient comprising the steps of:
- isolating stromal cells from infrapatellar fat pad tissue obtained from the patient without traditional expansion of the cells;
- enriching the non-expanded stromal cells for CD44 positive cells to provide a population of non-expanded chondro-progenitor cells;
- isolating a population of chondrocytes or chondrons from the patient without traditional expansion of the cells;
- seeding a tissue repair construct with the population of non-expanded chondro-progenitor cells and the population of non-expanded chondrocytes or chondrons to provide a bioactive tissue repair construct; and
- implanting the bioactive tissue repair construct into the defect site.

### Brief Description of the Figures

Fig. 1: The in vivo chondrogenic potential of freshly isolated CD44+ infrapatellar fat pad derived stromal cells (FPSCs) were compared to either fresh, unsorted FPSCs and culture expanded infrapatellar fat pad derived stem cells the gold standard). The comparison was performed in two different scaffolding systems, a fibrin hydrogel (this figure) and a cartilage ECM derived scaffold (see Figure 3 below). Data presented is after 28 days of subcutaneous implantation in a nude mice model.
Fig. 2: Controlled release of TGF-β3 from gelatin micropsheres loaded into fibrin hydrogels seeded with infrapatellar fat pad derived stromal cells promoted formation of cartilage *in vivo.* Biochemical analysis shows that freshly isolated CD44+ cells produce significantly higher levels of sGAG than both fresh (unsorted) and culture expanded infrapatellar fat pad derived stem cells groups.
Fig. 3: ECM derived scaffolds seeded with infrapatellar fat pad derived stromal cells and loaded with TGF-β3 promoted formation of cartilage *in vivo.* Superior sGAG (AB, alcian blue) accumulation for CD44+ cells (G) and expanded infrapatellar fat pad derived stem cell (E) seeded constructs. Stronger staining for collagen type two (Coll II) deposition (D) was observed for expanded cells when compared with the other groups,however CD44+ denoted more homogeneous and strong deposition (H) compared to fresh, unsorted cells (F).

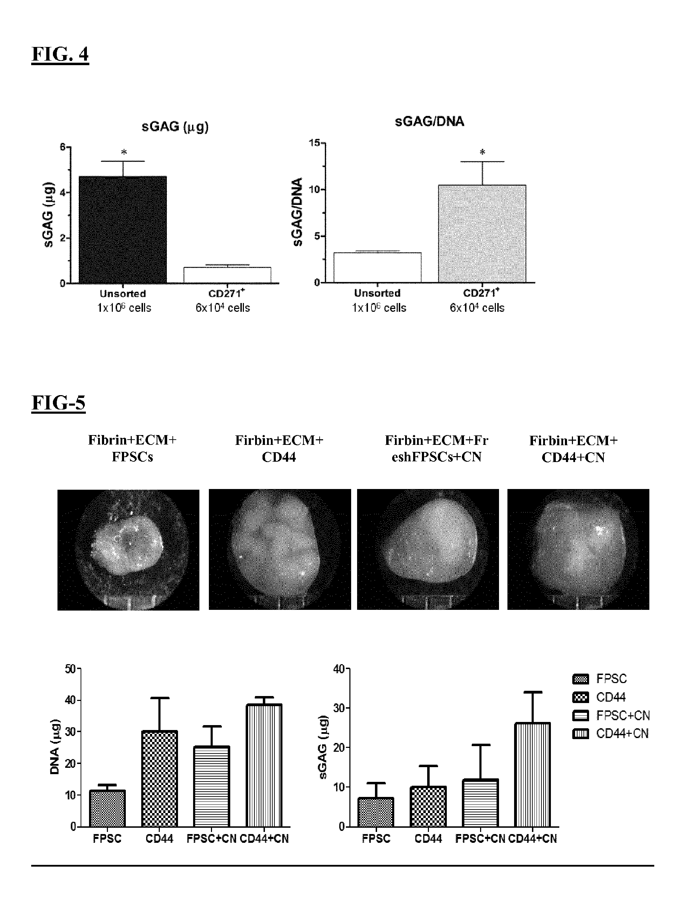

Fig. 4: ECM derived scaffolds seeded with unsorted freshly isolated infrapatellar fat pad derived stromal cells and CD271+ freshly isolated infrapatellar fat pad derived stromal cells. sGAG synthesis (sGAG/DNA) was higher for CD271+ cells compared to unsorted cells.
Fig. 5: Fibrin hydrogels containing cartilage ECM microparticles, chondrons and freshly isolated CD44+ infrapatellar fat pad derived stromal cells promoted the formation of cartilage in vivo. Higher levels of sGAG accumulation was observed with the combination of chondrons and CD44+ cells compared to all other groups.

### Detailed Description of the Invention

Broadly, the invention provides a non-expanded chondro-progenitor cell derived from adipose tissue of a mammal, particularly joint tissue adipose tissue, and ideally fat pad tissue. In particular, the invention provides a non-expanded chondro-progenitor cell derived from the infrapatellar fat pad of a mammal. The invention also relates to the use of the cells of the invention for *in-vivo* regeneration of tissue, especially cartilage tissue, and preferably articular cartilage tissue. In particular, the invention relates to the use of the cells of the invention for *in-vivo* articular cartilage regeneration. The cells of the invention is isolated by isolating stromal cells from adipose tissue without cell culture expansion, and then isolating cells from the non-expanded population of stromal cells that exhibit a cell surface marker associated with stem cells, for example CD44 or CD271, in which the isolated cells that exhibit the cell surface marker of stem cells are the non-expanded chondro-progenitor cells. The chondro-progenitor cells are then used for regenerating tissue, for example cartilage, and particularly articular cartilage, for example by seeding the cells (optionally in combination with other cell types, for example chondrocytes, or biological growth factors such as for example TGF-beta, or biological material such as for example extracellular matrix) into a solid, semi-solid, or liquid tissue repair construct, for example a scaffold or hydrogel, which is then implanted into an articular cartilage defect site. As the preparation of the cell population does not require cell culture expansion, the population may be prepared within 1-2 hours in a clinical setting, obviating the need for pre-culture of cells over a number of weeks and two surgical interventions.

The invention also provides an isolated population of non-expanded cells derived from adipose tissue of a mammal and enriched in chondro-progenitor cells. The non-expanded cells are preferably derived from joint adipose tissue, for example joint fat pad tissue and particularly infrapatellar pat pad tissue. The chondro-progenitor cells are positive for at least one cell surface marker of stem cells, for example CD44 or CD271, and ideally CD44. The invention also relates to the use of the population of cells of the invention for *in-vivo* regeneration of tissue, especially cartilage tissue, and preferably articular cartilage tissue. In particular, the invention relates to the use of the cells of the invention for *in-vivo* articular cartilage regeneration. The cell population of the invention is obtained by isolating stromal cells from adipose tissue, typically joint adipose tissue, and ideally fat pad tissue, without cell culture expansion, and then isolating cells that exhibit a cell surface marker associated with stem cells, for example CD44 or CD271 positive cells, from the non-expanded population of stromal cells, in which the cells that exhibit a cell surface marker associated with stem cells are the non-expanded chondro-progenitor cells. The population of chondro-progenitor cells is typicallyused for regenerating cartilage, especially articular cartilage, for example by seeding the cells (optionally in combination with other cell types, for example freshly isolated chondrocytes or chondrons, or biological growth factors such as for example TGF-beta, or biological material such as for example extracellular matrix, into a solid, semi-solid, or liquid matrix, for example a scaffold or hydrogel, which is then implanted into an articular cartilage defect site. As the preparation of the cell population does not require cell culture expansion, the population may be prepared within 1-2 hours in a clinical setting, obviating the need for pre-culture of cells over a number of weeks and two surgical interventions.

### Definitions

"Non-expanded" as applied to the cell or cell population of the invention should be understood to mean that the cell, cells or cell population are not cultured-expanded, and are therefore freshly-isolated cells.

"Chondro-progenitor" as applied to a cell should be understood to mean a cell that is predisposed to differentiate into a mature chondrocyte, and which expresses a cell surface marker associated with stem cells, especially CD44 and CD271.

"Cell surface marker associated with stem cells" as applied to a cell means a surface marker the presence of which indicates that the cell has stem cell qualities, i.e. the ability to differentiate into more than one cell lineage. Specific cell surface markers associated with stem cells, for the purpose of the presence invention, are selected from CD44, CD271, CD73, CD90, and CD105

"CD44 positive" as applied to a cell should be understood to mean that the cell expresses the CD44 surface biomarker. Methods for isolating cells that are CD44 positive are well described in the literature and commercial kits are available for isolating CD44 positive cells (MACS Mix Magnetic Beads, MACS Miltenyi Biotech).

"CD271 positive" as applied to a cell should be understood to mean that the cell expresses the CD271 surface biomarker. Methods for isolating cells that are CD271 positive are well described in the literature and commercial kits are available for isolating CD271 positive cells (MACS Mix Magnetic Beads, MACS Miltenyi Biotech).

"Chondrocyte" means a cartilage producing cell.

"Chondron" means a material comprising chondrocytes located within their own native territorial matrix. Methods for isolating chondrons are described in (Am J Sports Med 2013 41: 2158).

"Stromal cell" should be understood to mean a connective tissue cell of any organ.

"Adipose tissue" should be understood to mean loose tissue found in mammals that is composed mostly of adipocytes. Typically, the adipose tissue intended for the purpose of the present invention is joint adipose tissue (i.e. adipose tissue found in a joint), for example fat pad tissue, especially joint fat pad tissue, and particularly infrapatellar fat pad tissue found in the knee joint. However other sources of adipose tissue found in the mammalian body may be employed, for example subcutaneous fat, abdominal fat, ectopic fat or fat tissue derived from the shaft of long bones. The term should also be taken to include adipose tissue-associated synovial tissue or synovial membrane tissue.

"Derived from adipose tissue" as applied to cells means that the cells are isolated from adipose tissue or adipose-associated synovial tissue. Methods for isolating stromal cells from fat pad tissue, particularly the infrapatellar fat pad, are well known in the literature and described in, for example, Bekkers et al (Am J Sports Med 2013 41: 2158).

"Isolated population of non-expanded cells derived from adipose tissue and enriched in chondro-progenitor cells" should be understood to mean a population of non-expanded cells that are isolated from adipose tissue, especially joint adipose tissue, and are enriched in cells that exhibit a cell surface marker associated with stem cells, for example CD44 or CD271. The term "enriched in non-expanded chondro-progenitor cells" means that the proportion of non-expanded chondro-progenitor cells in the isolated population is greater than the proportion of chondro-progenitor cells in the stromal cell population from which the isolated population is derived. Other cell types may be present in the isolated population, for example carry-over adipocytes, vascular cells and fibroblasts.

"Isolating stromal cells from adipose tissue" means separating the stromal cells from their natural adipose tissue environment. Generally, the method involves comminuting the tissue, and then treating the comminuted tissue with collagenase to break down the ECM and release the cells which are isolated by, for example, centrifugation prior to filtering to isolate the stromal cells. Methods for isolation are described below and in Bekkers et al (Am J Sports Med 2013 41: 2158), Khan et al (Arthritis Research and Therapy 2008,10: R74), and Ye et al (PLOS One, June 11, 2014).

"Biological growth factor" means a naturally occurring substance capable of stimulating cellular growth, proliferation or differentiation. Examples of biological growth factors of the present invention include Fibroblast Growth Factor (i.e.FGF-2), Transforming Growth Factors (TGF-β1, TGF-β2 or TGF-β3), IFG, HGF, and cytokines IL-1, IL-2, IL-3, IL-4, IL-5, IL-6 and IL-7.

"Cartilage" should be understood to mean flexible connective tissue especially found in the joints and between bones of mammals that is composed of chondrocytes. "Articular cartilage" should be understood to mean a form of hyaline cartilage found at the articular end of joints.

"Extracellular matrix tissue" or "extracellular matrix" or "ECM" or "Extracellular matrix material" should be understood to mean a collection of extracellular molecules secreted by cells that provides structural and biochemical support to the surrounding cells. The ECM may be obtained from a mammal, for example a human or a non-human mammal, or it may be engineered in-vitro using published techniques, for example Vinardell et al (Vinardell, T., Sheehy, E., Buckley, C.T., Kelly, D.J. A comparison of the functionality and in vivo phenotypic stability of cartilaginous tissues engineered from different stem cells sources. Tissue Engineering Part A, 18(11-12), 1161-1170, 2012) and Buckley et al (Buckley, C.T., Vinardell, T., Kelly, D.J. Oxygen Tension Differentially Regulates the Functional Properties of Cartilaginous Tissues Engineered from Infrapatellar Fat Pad Derived MSCs and Articular Chondrocytes. Osteoarthritis and Cartilage, 18 (10), 1345-1354, 2010). Examples of extracellular matrix for the purpose of the present invention include cartilage ECM (obtained from porcine articular cartilage tissue) and growth plate ECM (typically obtained from the epiphysial plate of porcine tibia or femora).

"Hyaline cartilage ECM" should be understood to mean ECM obtained from hyaline cartilage which is a tissue found, for example, in the ear and nose and on joint surfaces. It is mostly composed of type II collagen and chondroitin sulphate. "Articular cartilage ECM" should be understood to mean ECM obtained from articular cartilage. "Growth plate ECM" or "growth plate tissue ECM" should be understood to mean ECM obtained from growth plate tissue of developing bones, typically developing long bones. This could include the epiphyseal plate in the metaphysis of a long bone, or articular cartilage from skeletally immature joints as this tissue is also known to act as a surface growth plate during development and skeletal maturation.

In this specification, the term "micronised" as applied to ECM should be understood to mean provided in a particulate form, in which the particles of ECM have a mean particle size of less than 200 microns as determined using routine light microscopy. Preferably, the micronised ECM has a mean particle size of less than 150 or 100 microns. Ideally, the micronized ECM has a mean particle size between 20 and 200 microns, 20 and 150 microns, 20 and 100 microns, 20 and 70 microns, 30 and 70 microns, 30 and 60 microns, 40 and 60 microns, and ideally about 50 microns. Methods of micronisation include grinding, milling, cryomilling. In this specification, the term "cryomilled" should be understood to mean a process in which a material is cryogenically frozen and then milled. Examples of cryomilling machines include the RETCH CRYOMILL™.

In this specification, the term "cells" should be understood to mean any type of cell, particularly stem cells, chondrocytes, and osteoblasts. Preferably, the cells are mesenchymal cells.

"Tissue repair construct" means a three dimensional construct (scaffold) having a solid or semi-solid 3-D matrix capable of being seeded with cells and is typically dimensioned for use in *in-vivo* repair of mammalian tissue, for example repair of cartilage, especially articular cartilage, defects. A "bioactive tissue repair construct" means a tissue repair construct that is seeded with cells or a cell population of the invention (or a composition of the invention). Examples of suitable constructs include solid scaffolds, for example a porous scaffold formed from micronized ECM, and hydrogels. Examples of tissue repair constructs for use with the present invention are described below and in the following literature documents:

Almeida, H.V., Liu, Y., Cunniffe, G., Buckley, C.T., Matsiko, A., O'Brien, F.J., Kelly, D.J. Controlled release of TGF-β3 from cartilage extra cellular matrix derived scaffolds to promote chondrogenesis of human joint tissue derived stem cells. Acta Biomaterilia (2014; doi: 10.1016/j.actbio.2014.05.030).

A biomimetic multi-layered collagen-based scaffold for osteochondral repair Levingstone, T.J., Matsiko, A., Dickson, G.R., O'Brien, F.J., Gleeson, J.P. Acta Biomaterialia 2014, 10 (5), pp. 1996-2004

In this specification, the term "seeding" as applied to a scaffold should be understood to mean incorporating a biological material into a scaffold. Method of seeding a scaffold include soaking the scaffold in a solution containing the biological material for a sufficient time to allow the biological material infiltrate the pores of the scaffold.

"Defect site in articular cartilage" should be understood to mean a region of the articular surface of a joint that has been damaged such that a loss of tissue has occurred. Examples of such defect sites include focal articular cartilage lesions.

"Patient" means a mammal, typically a human, and typically a human with a tissue defect, for example a cartilage defect, and usually a human with an articular cartilage defect. The therapeutic methods and uses of the invention are generally autologous cell based therapies, in which the cells or cell population of the invention that are used to treat a patient are obtained from the same patient. It will be appreciated that the cells are non-expanded cells.

### Experimental

### Isolation of fresh fat pad derived stromal cells and enriched stromal cells (CD44+/CD271+) for cartilage repair

Infrapatellar fat-pad derived stromal cells (FPSCs) were harvested under sterile conditions (after informed consent from the patients undergoing total knee replacement surgery). The fresh FPSCs isolation consists of two stages - tissue digestion by collagenase type II and rapid washing. The fat pad harvested from patients were weighed and minced into small pieces (∼ 2 - 3 mm) followed by two washes with phosphate buffer saline containing 2 % Penicillin/streptomycin. The small pieces of fat pad were then digested in 750 U/mL of collagenase type II (Life sciences) 4mL/ gram of tissue for 1-3 hrs. The concentration of collagenase can be increased and/or the tissue can be physically agitated or disrupted to reduce time required for tissue digestion. After this period, the collagenase activity was stopped by adding 20 % FBS containing DMEM media. The cell solution was sieved through a 100 µm cells strainer and centrifuged at 650 G for 5 mins. The residual pellet was triturated in fresh DMEM media containing 2 % Penicillin/streptomycin followed by sieving through a 40 µm cell strainer. The resulting cell solution was centrifuged at 650 g for 5 mins. The cell pellet was triturated in 20 mL DMEM containing 2% P/S. The CD44+ or CD271+ enriched cells were isolated from the freshly harvested cells solution using MACS mix magnetic beads by following the manufacturer protocol (MACS miltenyi Biotech).

### Development of decellularized ECM derived scaffolds with a uniform pore size.

Cartilage used in the fabrication of ECM derived scaffolds was harvested, in sterile conditions, from the femoral condyles of female pigs (3 months old) shortly after sacrifice. The cartilage was first broken up into small pieces using a scalpel. Cartilage particles were then broken up using a cryogenic mill (6770 Freezer/Mill, SPEX, UK). Before or after particulation of the cartilage tissue it can be decellularized using chemical techniques. These small pieces of cartilage where then homogenized in distilled water (dH2O) using a homogenizer (IKAT10, IKA Works Inc, NC, USA) to create a cartilage slurry (250 mg/ml). The slurry was transferred to custom made moulds (containing wells 5 mm in diameter and 3 mm in height) and freeze-dried (FreeZone Triad, Labconco, KC, USA) to produce porous scaffolds. Briefly, the slurry was frozen to -30°C (1°C/min) and kept at that temperature for one hour. The temperature was then increased to -10°C (1°C/min), followed by a hold period of 18-24 hours and then finally increased to room temperature (0.5°C/min). Next, two different crosslinking techniques were applied to the scaffolds. The scaffolds underwent DHT and 1-Ethyl-3-3dimethyl aminopropyl carbodiimide (EDAC) crosslinking. The DHT process was performed in a vacuum oven (VD23, Binder, Germany), at 115°C, in 2 mbar for 24 hours. The EDAC (Sigma-Aldrich, Germany) crosslinking consisted of chemical exposure for 2 hours at a concentration of 6 mM in the presence of N-Hydroxysuccinimide (NHS) (Sigma-Aldrich, Germany). A molar ratio of 2.5 M EDAC/M N-Hydroxysuccinimide was used. After EDAC crosslinking the scaffolds were washed twice in sterile PBS (Sigma-Aldrich, Germany).

### Fabrication and seeding offibrin-cartilage ECM composite hydrogels

Fabrication of the fibrin hydrogel and encapsulation of the FPSCs is performed at the same time once the fibrinogen is combined with thrombin. ECM particles are produced using the cryomilling procedure described above. Fibrin-ECM composite hydrogels are fabricated by mixing devitalized or decellularised particulated porcine cartilage (1.5% w/v) ECM with fibrinogen (100 mg/ml). If the aim is to also incorporate TGF-β3 into the construct, the particulated ECM is first soak loaded with the desired amount of growth factor. This suspension is mixed with FPSCs, and then hydrogels are formed by adding this mixture to appropriately shaped moulds and then adding thrombin. Control constructs consist of fibrin hydrogels instead loaded with gelatin microspheres (MS). Following gelation, cell seeded hydrogels were implanted subcutaneously into the back of nude mice.

### Seeding of scaffolds

Each scaffold is ideally seeded with between 50,000 and 500,000 human FPSCs. For *in vivo* studies, scaffolds were implanted subcutaneously into the back of nude mice immediately following cell seeding. For *in vitro* experiments, scaffolds were maintained in chemically defined chondrogenic medium (CDM), as previously described, for 28 days (at 5% O₂ and 37°C). CDM consists of DMEM GlutaMAXTM supplemented with penicillin (100 U/ml)-streptomycin (100 µg/ml) (both GIBCO, Biosciences, Ireland), 100 µg/ml sodium pyruvate, 40 µg/ml L-proline, 50 µg/ml L-ascorbic acid-2-phosphate, 1.5 mg/ml BSA, 1x insulin-transferrin-selenium, 100 nM dexamethasone (all from Sigma-Aldrich, Ireland) and 10 ng/ml recombinant human growth factor-β3 (TGF-β3; ProSpec-Tany TechnoGene Ltd, Israel). The scaffolds are kept in 12 well plates and each scaffold is placed within cylindrical agarose moulds (slightly larger than the scaffold) to prevent cell migration into the culture wells. After seeding, the scaffolds with the cells plus 40 µL of CDM are left in the incubator for two hours. After two hours, 2.5 ml of supplemented CDM are added to each well. Media changes are performed twice a week.

### Composition comprising fad pad derived enriched cells + chonrons, and use (prophetic example ?)

Chondrons from cartilage were harvested under sterile conditions (after informed consent from the patients undergoing total knee replacement surgery). The fresh chondron isolation consists of two stages - tissue digestion by collagenase and repeated washing. The cartilage harvested from patients were weighted and minced into small pieces (∼ 2 - 3 mm) followed by two washes with phosphate buffer saline containing 2 % Penicillin/streptomycin. The small pieces of cartilage were then digested in 350 U/mL of collagenase type II (8mL/ gram) of tissue for 2 hrs. The concentration of collagenase can be increased and/or the tissue can be physically agitated or disrupted to reduce time required for tissue digestion. After this period, the collagenase activity was stopped by adding 20 % FBS containing DMEM media. The cell solution was sieved through a 100 µm cells strainer and centrifuged at 650 G for 5 mins. The residual pellet was triturated in fresh DMEM media containing 2 % Penicillin/Streptomycin (P/S) followed by sieving through a 40 µm cell strainer. The resulting cell solution was centrifuged at 650 g for 5 mins. The cell pellet was triturated in 20 mL DMEM containing 2% P/S. For the co-culture groups, freshly isolated cells were mixed with fibrin/ECM constructs using 25:75 ratio of chondrons to freshly isolated FPSCs (CD44+ or CD271+). The formed cell encapsulated constructs were implanted subcutaneously in mice (A subcutaneous incision is made in the back of the mice. A small pocket is made underneath the skin of the mouse, into which the engineered constructs are placed).

### Results

The chondrogenic potential of fresh CD44+ cells were compared to either fresh, unsorted FPSCs and culture expanded FPSCs (the gold standard). The comparison was performed in two different scaffolding systems, a fibrin hydrogel and a cartilage ECM derived scaffold. Macroscopically, the cartilage tissue produced using freshly isolated CD44+ cells seeded in the fibrin hydrogel is similar to that generated using culture expanded cells (Fig. 1)

Controlled release of TGF-β3 from gelatin micropsheres loaded into fibrin hydrogels seeded with stem cells promoted formation of cartilage *in vivo.* Biochemical analysis shows that fresh CD44+ cells have significantly higher sGAG synthesis than both fresh (unsorted) and expanded FPSC groups (Fig. 2).

ECM derived scaffolds seeded with infrapatellar fat pad derived stromal cells and loaded with TGF-β3 promoted formation of cartilage *in vivo.* Comparable sGAG (AB, alcian blue) accumulation was observed for CD44+ cells (G) and expanded cells (E). Collagen type two (Coll II) synthesis for the expanded cells was superior when compared with the other groups, however CD44+ cells stained stronger (H) compared to fresh unsorted cells (Fig. 3).

ECM derived scaffolds seeded with unsorted freshly isolated infrapatellar fat pad derived stromal cells and CD271+ freshly isolated infrapatellar fat pad derived stromal cells. sGAG synthesis (sGAG/DNA) was higher for CD271+ cells compared to unsorted cells (Fig. 4).

ECM derived particles combined with a co-culture of freshly isolated CD44+ FPSCs and chondrons enhanced the formation of cartilage specific proteoglycans (GAG) *in vivo* (Fig 5).

The invention is not limited to the embodiments hereinbefore described which may be varied in construction and detail without departing from the spirit of the invention.

## Claims

1. An isolated population of non-expanded cells derived from adipose tissue of a mammal and enriched in non-expanded chondro-progenitor cells.

2. A isolated population of non-expanded cells according to Claim 1 wherein the non-expanded chondroprogenitor cells are CD44 or CD271 positive.

3. A isolated population of non-expanded cells according to Claim 1 or 2 that are derived from an infrapatellar fat pad of a mammal, wherein the non-expanded chondroprogenitor cells are CD44 or CD271 positive.

4. An isolated population of non-expanded cells of Claim 1 obtained by the steps of isolating stromal cells from adipose tissue without culture expansion of the cells, enriching the non-expanded stromal cells for cells that exhibit a sell surface marker associated with stem cells to provide a population of non-expanded chondro-progenitor cells.

5. An isolated population of non-expanded cells of Claim 2 obtained by the steps of isolating stromal cells from adipose tissue without culture expansion of the cells, and enriching the non-expanded stromal cells for cells that are CD44 or CD271 positive to provide a population of non-expanded chondro-progenitor cells.

6. A composition comprising an isolated population of non-expanded cells of any of Claims 1 to 5 from a patient in combination with freshly isolated chondrocytes or chondrons from the same patient.

7. A composition comprising an isolated population of non-expanded cells of any of Claims 1 to 5 and micronized extracellular matrix.

8. A bioactive tissue repair construct comprising a tissue repair construct seeded with an isolated population of non-expanded cells of any of Claims 1 to 5.

9. A bioactive tissue repair construct as claimed in Claim 8 for use in a method of autologous cell-based *in-vivo* repair of a cartilage defect site in a patient.

10. An isolated population of non-expanded cells of any of Claims 1 to 5 for use in a method of autologous cell-based therapy of a cartilage defect site in a patient.

11. A method of isolating a population of non-expanded adipose tissue derived chondro-progenitor cells comprising the steps of isolating stromal cells from adipose tissue without expansion of the cells, enriching the non-expanded stromal cells for cells that exhibit a cell surface marker associated with stem cells to provide a population of non-expanded chondro-progenitor cells.

12. A method as claimed in Claim 11 in which the non-expanded stromal cells are enriched for cells that are CD44 or CD271 positive to provide the population of non-expanded chondro-progenitor cells.

13. A method as claimed in Claim 11 or 12 in which the stromal cells are isolated from joint fat pad tissue.

14. A freshly isolated CD44+ve or CD271+ve mammalian adipose tissue-derived stromal cell.

15. A freshly isolated CD44+ve or CD271+ve mammalian tissue-derived stromal cell according to Claim 14 that is derived from mammalian infrapatellar fat pad tissue.
